# EUROPÄISCHE PATENTSCHRIFT

(11) **EP 2 304 106 B1**
(45) Veröffentlichungstag und Bekanntmachung des Hinweises auf die Patenterteilung: **12.09.2018**
(21) Anmeldenummer: 09769123.2
(22) Anmeldetag: 09.06.2009
(51) Int. Cl.: D21H 21/20, D21H 17/55, D21H 17/36, D21H 17/37, D21H 23/04

(54) **HERSTELLUNG VON PAPIER**
PRODUCTION OF PAPER
PRODUCTION DE PAPIER

(30) Priorität: 24.06.2008 EP 08158838
(43) Veröffentlichungstag der Anmeldung: 06.04.2011
(73) Patentinhaber: BASF SE, 67056 Ludwigshafen am Rhein (DE)
(72) Erfinder: HÄHNLE, Hans-Joachim, 67435 Neustadt (DE); ESSER, Anton, 67117 Limburgerhof (DE)
(74) Vertreter: BASF IP Association
(86) Internationale Anmeldenummer: PCT/EP2009/057104
(87) Internationale Veröffentlichungsnummer: WO 2009/156274

(56) Entgegenhaltungen:
- EP-A- 0 374 646
- WO-A-02/101144
- WO-A-2005/012637
- WO-A-2005/059251
- WO-A-2005/089175
- WO-A-2006/010268
- DE-A1- 4 241 117
- DE-A1- 19 526 626
- US-A- 5 630 907
- US-A1- 2006 037 727

## Beschreibung

Die Erfindung betrifft die Verwendung von amphoteren, Amidingruppen enthaltenden Copolymeren als Mittel zur Erhöhung der initialen Nassgefügefestigkeit von Papier.

Unter initialer Nassgefügefestigkeit (engl.: initial wet web strength) wird die Festigkeit eines nassen Papiers verstanden, das niemals getrocknet wurde. Es handelt sich hierbei um die Festigkeit eines nassen Papiers, wie es bei der Papierherstellung nach Durchlaufen der Sieb- und Pressenpartie der Papiermaschine vorliegt. Es enthält typischer Weise ca. 50 % Wasser.

Von der initialen Nassgefügefestigkeit zu unterscheiden sind die Nassfestigkeit und die initiale Nassfestigkeit von Papier, weil beide Eigenschaften an Papieren gemessen werden, die nach dem Trocknen wieder auf einen definierten Wassergehalt angefeuchtet werden. Die initiale Nassfestigkeit ist ein wichtiger Parameter bei der Beurteilung von nicht permanent nassfesten Papieren. Ein getrocknetes und danach wieder angefeuchtetes Papier hat eine ganz andere Nassfestigkeit als ein feuchtes Papier, das direkt nach dem Durchlaufen der Sieb- und Pressenpartie einer Papiermaschine vorliegt. Eine ausführliche Beschreibung der Initialen Nassgefügefestigkeit und ihrer Bedeutung in der Papierherstellung geben M. Schwarz und K. Bechtel in dem Beitrag "Initiale Gefügefestigkeit bei der Blattbildung" in Wochenblatt für Papierfabrikation 131, Seiten 950 - 957 (2003) Nr. 16.

Ein entscheidend limitierender Faktor auf dem Weg zu einer weiteren Steigerung der Geschwindigkeit von Papiermaschinen ist die initiale Nassgefügefestigkeit. Sie begrenzt die maximal anwendbare Kraft, die auf ein gerade in der Papiermaschine gebildetes Blatt ausgeübt werden kann, das die Sieb- und die Pressenpartie der Maschine passiert hat und in die Trockenpartie übergeben wird. Hierbei muss das Blatt von den Presswalzen abgezogen werden. Um einen abrissfreien Betrieb einer Papiermaschine sicher gewährleisten zu können, muss an dieser Stelle die angewendete Abzugskraft deutlich kleiner sein als die initiale Nassgefügefestigkeit des feuchten Papiers. Eine Erhöhung der initialen Nassgefügefestigkeit erlaubt die Anwendung höherer Abzugskräfte und damit ein schnelleres Betreiben der Papiermaschine, vgl. EP-B-0 780 513.

Es ist zwar bekannt, dass die initiale Nassgefügefestigkeit dadurch gesteigert werden kann, dass der Festgehalt des Papiers an der Stelle zwischen Press- und Trockenpartie im Herstellprozess erhöht wird. Inzwischen sind jedoch maschinentechnisch weitgehend alle Möglichkeiten ausgereizt, eine weitere Erhöhung der initialen Nassgefügefestigkeit zu erreichen. Auch der Möglichkeit, den Festgehalt an dieser Stelle des Prozesses durch Additive zur Steigerung der Entwässerung zu verbessern, sind Grenzen gesetzt, weil gleichzeitig eine gute Formation des sich bildenden Blattes gewährleistet werden muss.

Bisher ist kein Verfahren beschrieben, mit dem die initiale Nassgefügefestigkeit von Papier durch Zusatz eines Additivs ohne Steigerung des Festgehaltes direkt beeinflusst werden kann.

WO-A-04/087818, WO-A-05/012637 und WO-A-2006066769 beschreiben wässrige Anschlämmungen von feinteiligen Füllstoffen, die zumindest teilweise mit Polymerisaten überzogen sind und die erhältlich sind durch Behandeln von wässrigen Anschlämmungen feinteiliger Füllstoffe mit mindestens einem wasserlöslichen amphoteren Copolymerisat, das 6-Ring-Amidine enthält. Diese Anschlämmungen ermöglichen eine Erhöhung des Füllstoffgehalts in Papieren unter Erhalt der Papiereigenschaften insbesondere der Trockenfestigkeit.

Aus den älteren Anmeldungen EP 07 111 859.0 und EP 07 111 617.2 ist außerdem bekannt, den Füllstoffgehalt von Papier dadurch zu erhöhen, dass man Füllstoffe vor dem Einsatz im Papierherstellungsprozeß mit den vorstehend genannten Polymeren vorbehandelt, wobei die Vorbehandlung zusätzlich in Gegenwart von gequollener Stärke bzw. zusätzlich in Gegenwart von Latices vorgenommen wird.

Aus der JP-A 08059740 ist bekannt, dass man zu wässrigen Suspensionen von anorganischen Teilchen amphotere wasserlösliche Polymere zusetzt, wobei zumindest ein Teil der Polymeren auf der Füllstoffoberfläche adsorbiert wird. Die amphoteren Polymeren werden vorzugsweise durch Hydrolysieren von Copolymerisaten aus N-Vinylformamid, Acrylnitril und Acrylsäure in Gegenwart von Säuren hergestellt. Sie enthalten 20 bis 90 Mol-% 5-Ring Amidineinheiten der Struktur 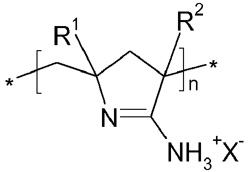 in der R¹ und R² jeweils H oder eine Methylgruppe, n eine ganze Zahl und X ein Anion bedeuten. Die mit solchen Polymeren behandelten Füllstoffslurries werden bei der Herstellung von füllstoffhaltigen Papieren dem Papierstoff zugesetzt. Die Füllstoffbehandlung führt zu einer Verbesserung der Entwässerung des Papierstoffs und ergibt außerdem eine Verbesserung verschiedener Festigkeitseigenschaften des getrockneten Papiers sowie eine Verbesserung der Füllstoffretention.

Weiterhin werden in EP-A-0528409 und in DE-A-4328975 schwach amphotere Polymere beschrieben, die 5-Ring-Amidine enthalten. Im ersteren Fall werden sie als Flockungsmittel eingesetzt, während sie im zweiten Fall als Additive zur Papierherstellung Anwendung finden. Allerdings wird in beiden Anmeldungen darauf hingewiesen, dass der Anteil der anionischen Struktureinheiten für die Wirksamkeit schädlich ist und daher typischerweise kleiner als 5 mol% betragen soll, vgl. EP-A-0528409, Seite 5, Zeile 41f und DE-A-4328975, Seite 6, Abschnitt 0027.

In keiner der genannten Veröffentlichungen wird die Beeinflussung der initialen Nassgefügefestigkeit durch Verwendung von amphoteren, Amidineinheiten enthaltenden Polymeren bei der Papierherstellung erwähnt.

US 5630907 bzw. die korrespondierende DE-A-4241117 beschreibt die Verwendung von Copolymerisaten, die durch Copolymerisieren von N-Vinylcarbonsäureamiden mit anderen monoethylenisch ungesättigten Verbindungen und teilweise oder vollständige Abspaltung der Formylgruppen aus dem in das Copolymerisat einpolymerisierten N-Vinylcarbonsäureamiden unter Bildung von Amin- bwz. Ammoniumgruppen erhältlich sind, als Zusatz zum Papierstoff zur Erhöhung der Entwässerungsgeschwindigkeit und der Retention bei der Papierherstellung sowie der Trocken- und Naßfestigkeit des Papiers. Eine Zugabe zu einem Dickstoff mit einer Faserkonzentration von >15 g/l wird nicht genannt.

DE-A-19526626 beschreibt Propfpolymerisate aus Vinylester- und/oder Vinylalkohol-Einheiten enthaltenden Polymerisaten und offenkettigen N-Vinylcarbonsäureamiden, Verfahren zu ihrer Herstellung und die Verwendung der Pfropfpolymerisate unter anderem bei der Herstellung von Papier, Pappe und Karton. Eine Zugabe zu einem Dickstoff mit einer Faserkonzentration von >15g/l wird nicht genannt.

Der Erfindung liegt die Aufgabe zugrunde, bei der Herstellung von Papier die initiale Nassgefügefestigkeit der noch feuchten Paperbahn vor dem Übergang in die Trockenpartie zu erhöhen, um gegenüber bekannten Verfahren im Papierherstellungsprozeß höhere Maschinengeschwindigkeiten zu erreichen.

Die Aufgabe wird erfindungsgemäß gelöst mit der Verwendung von wasserlöslichen, amphoteren Copolymeren, die erhältlich sind durch durch Copolymerisieren von
a) wenigstens einem N-Vinylcarbonsäureamid der allgemeinen Formel 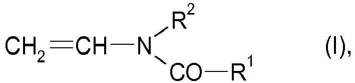 worin R¹ und R² unabhängig voneinander für H oder C₁- bis C₆-Alkyl stehen,
b) wenigstens einem Monomer, das ausgewählt ist aus der Gruppe bestehend aus
   (b1) monoethylenisch ungesättigten Sulfonsäuren, Phosphonsäuren, Phosphorsäureestern und Derivaten davon, und
   (b2) monoethylenisch ungesättigten Mono- und Dicarbonsäuren, deren Salzen und Dicarbonsäureanhydriden,
c) gegebenenfalls wenigstens einem von den Komponenten (a) und (b) verschiedenen monoethylenisch ungesättigten Monomer, und
d) gegebenenfalls wenigstens einer Verbindung, die mindestens zwei ethylenisch ungesättigte Doppelbindungen im Molekül aufweist,
mit der Maßgabe, dass das Monomerengemisch mindestens ein Monomer (b) mit mindestens einer freien Säuregruppe und/oder eine Säuregruppe in Salzform enthält,
und anschließende teilweise oder vollständige Hydrolyse der Gruppen -CO-R¹ aus den in das Copolymerisat einpolymerisierten Monomeren (a), und diese Copolymere
(i) 5 bis 70 Mol-% Vinylcarbonsäureamideinheiten,
(ii) 5 bis 45 Mol-% Einheiten von Acrylsäure, Methacrylsäure, Salzen und Gemischen davon,
   und
(iii) 10 bis 60 Mol-% Vinylamineinheiten der Formel VI in Salzform 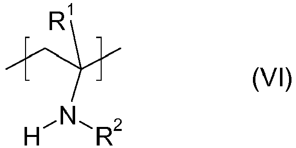 und/oder Amidineinheiten der Formel (II) und/oder (III) 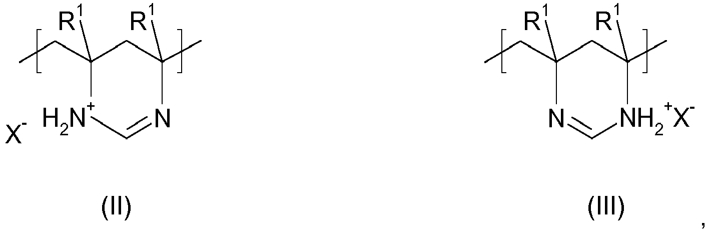 wobei in den Amidineinheiten (II) und (III) X-jeweils ein Anion bedeutet und die Substituenten R¹ und R² in den Formeln II, III und VI jeweils die in Formel I angegebene Bedeutung haben, enthalten,
als Mittel zur Erhöhung der initialen Nassgefügefestigkeit von Papier,
dadurch gekennzeichnet, dass die wasserlöslichen, amphoteren Copolymeren in einer Menge von 0,03 bis 1,00 Gew.-%, bezogen auf trockenen Faserstoff, einem Dickstoff mit einer Faserkonzentration von > 15 g/l zugesetzt werden.

Die Behandlung der Fasern erfolgt im Dickstoff im Papierherstellungsprozeß. Ein Dickstoff hat eine Faserkonzentration von >15 g/l, z.B. in dem Bereich von 25 bis 40 g/l bis zu 60 g/l, während ein Dünnstoff z.B. eine Faserkonzentration von <15 g/l, z.B. in dem Bereich von 5 bis 12 g/l hat.

Bei den hydrolysierten Copolymeren beträgt beispielsweise das Verhältnis A von Amidineinheiten zu Amineinheiten 100 : 1 bis 1 : 30, bevorzugt 40 : 1 bis 1 :15, besonders bevorzugt 8 : 1 bis 1 : 8. Das Verhältnis B von kationischen zu anionischen Einheiten liegt z. B. in dem Bereich von 20 : 1 bis 1 : 20, bevorzugt 12:1 bis 1 : 12, besonders bevorzugt 7:1 bis 1 : 7. Unter kationischen Einheiten ist in diesem Zusammenhang die Summe aus Amin- und Amidineinheiten zu verstehen, während unter anionischen Einheiten die Säureeinheiten subsummiert werden, die bei der Copolymerisation aus den Monomeren der Gruppe (b) entstehen und die in Form der freien Säuregruppen und/oder in Salzform vorliegen.

Die nicht hydrolysierten Copolymere enthalten jeweils wenigstens ein Monomer der Gruppen (a) und (b) sowie gegebenenfalls wenigstens ein Monomer der Gruppe (c) und gegebenenfalls wenigstens ein Monomer der Gruppe (d) in einpolymerisierter Form.

Beispiele für Monomere der Gruppe (a) sind offenkettige N-Vinylamidverbindungen der Formel (I) wie beispielsweise N-Vinylformamid, N-Vinyl-N-methylformamid, N-Vinylacetamid, N-Vinyl-N-methylacetamid, N-Vinyl-N-ethylacetamid, N-Vinylpropionamid und N-Vinyl-N-methylpropionamid und N-Vinylbutyramid. Die Monomeren der Gruppe (a) können allein oder in Mischung bei der Copolymerisation mit den Monomeren der anderen Gruppen eingesetzt werden. Aus dieser Gruppe wird vorzugsweise N-Vinylformamid bei der Copolymerisation eingesetzt.

Die erfindungsgemäß einzusetzenden Copolymere enthalten wenigstens ein Monomer der Gruppe (b), wobei diese ausgewählt sind aus der Gruppe bestehend aus
(b1) monoethylenisch ungesättigten Sulfonsäuren, Phosphonsäuren, Phosphorsäureestern und Derivaten davon, und
(b2) monoethylenisch ungesättigten Mono- und Dicarbonsäuren, deren Salzen und Dicarbonsäureanhydriden.

Als Monomere der Gruppe (b1) sind Verbindungen geeignet, die einen organischen Rest mit einer polymerisierbaren, α,β-ethylenisch ungesättigten Doppelbindung und mindestens einer Sulfonsäure- oder Phosphonsäuregruppe pro Molekül aufweisen. Geeignet sind weiterhin die Salze und Ester der zuvor genannten Verbindungen. Bei den Estern der Phosphonsäuren kann es sich dabei um die Mono- oder die Diester handeln. Geeignete Monomere (b1) sind weiterhin Ester der Phosphorsäure mit Alkoholen mit einer polymersierbaren, α,β-ethylenisch ungesättigten Doppelbindung. Dabei kann eines oder können die beiden übrigen Protonen der Phosphorsäuregruppe durch geeignete Basen neutralisiert oder mit Alkoholen, die keine polymerisierbaren Doppelbindungen aufweisen, verestert werden.

Geeignete Basen zur teilweisen oder vollständigen Neutralisation der Säuregruppen der Monomere (b1) sind beispielsweise Alkalimetall- oder Erdalkalimetallbasen, Ammoniak, Amine und/oder Alkanolamine. Beispiele hierfür sind Natriumhydroxid, Kaliumhydroxid, Natriumcarbonat, Kaliumcarbonat, Natriumhydrogencarbonat, Kaliumhydrogencarbonat, Magnesiumhydroxid, Magnesiumoxid, Calciumhydroxid, Calciumoxid, Triethanolamin, Ethanolamin, Morpholin, Diethylentriamin oder Tetraethylenpentamin. Geeignete Alkohole zur Veresterung der Phosphorsäure sind beispielsweise C₁-C₆-Alkanole, wie beispielsweise Methanol, Ethanol, n-Propanol, Isopropanol, n-Butanol, sec-Butanol, tert.-Butanol, n-Pentanol, n-Hexanol sowie deren Isomere.

Zu den Monomeren (b1) zählen beispielsweise Vinylsulfonsäure, Allylsulfonsäure, Methallylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, Acrylamidomethylenphosphonsäure, 2-Acrylamido-2-methylpropansulfonsäure, Vinylphosphonsäure, CH₂=CH-NH-CH₂-PO₃H, Vinylphosphonsäuremonomethylester, Vinylphosphonsäuredimethylester, Allylphosphonsäure, Allylphosphonsäuremonomethylester, Allylphosphonsäuredimethylester, Acrylamidomethylpropylphosphonsäure, (Meth)acrylethylenglykolphosphat und Phosphorsäuremonoallylester.

Werden als Komponente (b1) ausschließlich Monomere eingesetzt, bei denen alle Protonen der Säuregruppen verestert sind, wie z.B. Vinylphosphonsäuredimethylester oder Allylphosphonsäuredimethylester, so wird zur Polymerisation wenigstens eine monoethylenisch ungesättigte Mono- und/oder Dicarbonsäure oder ein Salz davon eingesetzt, wie sie im Folgenden als Komponente (b2) beschrieben werden. Somit ist sichergestellt, dass die erfindungsgemäß eingesetzten Copolymerisate anionogene/anionische Gruppen aufweisen. Alternativ dazu können auch die Bedingungen für die Hydrolyse so gewählt werden, dass die Estergruppen teilweise unter Bildung von Säuregruppen im Copolymer hydrolysiert werden.

Die zuvor genannten Monomere (b1) können einzeln oder in Form von beliebigen Mischungen eingesetzt werden.

Als Monomere der Gruppe (b2) kommen z.B. monoethylenisch ungesättigte Carbonsäuren mit 3 bis 8 C-Atomen sowie die wasserlöslichen Salze wie Akalimetall-, Erdalkalimetall- oder Ammoniumsalze dieser Carbonsäuren und die monoethylenisch ungesättigten Carbonsäureanhydride in Betracht. Zu dieser Gruppe von Monomeren gehören beispielsweise Acrylsäure, Methacrylsäure, Dimethacrylsäure, Ethacrylsäure, α-Chloracrylsäure, Maleinsäure, Maleinsäureanhydrid, Fumarsäure, Itoconsäure, Mesaconsäure, Citraconsäure, Glutaconsäure, Aconitsäure, Methylenmalonsäure, Allylessigsäure, Vinylessigsäure und Crotonsäure. Die Monomeren dieser Gruppe (b2) können allein oder in Mischung miteinander, in teilweise oder in vollständig neutralisierter Form bei der Copolymerisation eingesetzt werden. Zur Neutralisation geeignete Basen sind die bei der Komponente (b1) genannten.

Das wasserlösliche amphotere Copolymerisat enthält wenigstens ein Monomer aus der Gruppe (b), das ausgewählt ist aus den Untergruppen (b1) und (b2) einpolymerisiert. Selbstverständlich kann das wasserlösliche amphotere Copolymerisat auch Mischungen von Monomereinheiten aus den Untergruppen (b1) und (b2) enthalten.

Die Copolymerisate können zur Modifizierung gegebenenfalls wenigstens ein weiteres Monomer der Gruppe (c) in einpolymerisierter Form enthalten. Vorzugsweise sind diese Monomere Nitrile von α,β-ethylenisch ungesättigten Mono- und Dicarbonsäuren, wie beispielsweise Acrylnitril und Methacrylnitril. Bei der Hydrolyse solcher Copolymerisate werden dann 5 Ring-Amidine erhalten.

Weiterhin geeignete Monomere der Gruppe (c) sind:
Ester α,β-ethylenisch ungesättigter Mono- und Dicarbonsäuren mit einwertigen C₁-C₃₀-Alkanolen, C₂-C₃₀-Alkandiolen und C₂-C₃₀-Aminoalkoholen, Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, N-Vinyllactamen, stickstoffhaltigen Heterocyclen und Lactone mit α,β-ethylenisch ungesättigten Doppelbindungen, Vinylaromaten, Vinylhalogeniden, Vinylidenhalogeniden, C₂-C₈-Monoolefinen und Mischungen davon.

Beispiele für Vertreter dieser Gruppe (c) sind z.B. Methyl(meth)acrylat (die Formulierung "...(meth)acrylat" bedeutet jeweils "...methacrylat" als auch "...acrylat"), Methylethacrylat, Ethyl(meth)acrylat, Ethylethacrylat, n-Butyl(meth)acrylat, Isobutyl(meth)acrylat, tert.-Butyl(meth)acrylat, tert.-Butylethacrylat, n-Ocytl(meth)acrylat, 1,1,3,3-Tetramethylbutyl(meth)acrylat, Ethylhexyl(meth)acrylat und Mischungen davon.

Geeignete zusätzliche Monomere (c) sind weiterhin die Ester von α,β-ethylenisch ungesättigten Mono- und Dicarbonsäuren mit Aminoalkoholen, vorzugsweise C₂-C₁₂-Aminoalkoholen. Diese können am Aminstickstoff C₁-C₈-monoalkyliert oder -dialkyliert sein. Als Säurekomponente dieser Ester eignen sich z.B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Dazu zählen beispielsweise N-Methylaminomethyl(meth)acrylat, N-Methylaminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat.

Geeignete zusätzliche Monomere (c) sind weiterhin Acrylsäureamid, Methacrylsäureamid, N-Methyl(meth)acrylamid (die Formulierung "...(meth)acrylamid" steht jeweils für "...acrylamid" sowie für "...methacrylamid"), N-Ethyl(meth)acrylamid, n-Propyl(meth)acrylamid, N-(n-Butyl)(meth)acrylamid, tert.-Butyl(meth)acrylamid, n-Octyl(meth)acrylamid, 1,1,3,3-Tetramethylbutyl(meth)acrylamid, Ethylhexyl(meth)acrylamid und Mischungen davon.

Weiterhin sind als Monomere (c) geeignet 2-Hydroxyethyl(meth)acrylat, 2-Hydroxyethylethacrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat und Mischungen davon.

Darüber hinaus sind als weitere Monomere (c) N-[2-(Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[2-(Diethylamino)ethyl]methacrylamid und Mischungen davon geeignet.

Geeignete Monomere (c) sind weiterhin N-Vinyllactame und deren Derivate, die z.B. einen oder mehrere C₁-C₆-Alkylsubstituenten (wie oben definiert) aufweisen können. Dazu zählen N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam und deren Mischungen.

Weiterhin sind als Monomere (c) N-Vinylimidazole und Alkylvinylimidazole geeignet, insbesondere Methylvinylimidazole wie beispielsweise 1-Vinyl-2-methylimidazol, 3-Vinylimidazol-N-oxid, 2- und 4-Vinylpyridin-N-oxide sowie betainische Derivate und Quaternisierungsprodukte dieser Monomere.

Geeignete zusätzliche Monomere sind weiterhin Ethylen, Propylen, Isobutylen, Butadien, Styrol, α-Methylstyrol, Vinylacetat, Vinylpropionat, Vinylchlorid, Vinylidenchlorid, Vinylfluorid, Vinylidenfluorid und Mischungen davon.

Die zuvor genannten Monomere (c) können einzeln oder in Form von beliebigen Mischungen eingesetzt werden.

Eine weitere Modifizierung der Copolymerisate ist dadurch möglich, dass man bei der Copolymerisation Monomere (d) einsetzt, die mindestens zwei Doppelbindungen im Molekül enthalten, z.B. Triallylamin, Methylenbisacrylamid, Glykoldiacrylat, Glykoldimethacrylat, Glycerintriacrylat, Pentaerythrittriallylether, mindestens zweifach mit Acrylsäure und/oder Methacrylsäure veresterte Polyalkylenglykole oder Polyole wie Pentaerythrit, Sobit oder Glukose. Ebenfalls geeignet sind Allyl und Vinylether von Polyalkylenglykole oder Polyole wie Pentaerythrit, Sobit oder Glukose. Falls mindestens ein Monomer der Gruppe (d) bei der Copolymerisation eingesetzt wird, so betragen die angewendeten Mengen bis zu 2 Mol-%, z.B. 0,001 bis 1 Mol-%.

In einer bevorzugten Ausführungsform wird zur Polymerisation ein Monomergemisch eingesetzt, wobei die Komponente (b) entweder nur aus Monomeren (b1) oder nur aus Monomeren der Untergruppe (b2) besteht, mit der Maßgabe, dass das Monomerengemisch mindestens ein Monomer (b) mit mindestens einer freien Säuregruppe und/oder einer Säuregruppe in Salzform enthält.

In einer besonders bevorzugten Ausführungsform werden zur Polymerisation mit den Monomeren (a) nur Monomere der Untergruppe (b2) eingesetzt.

Beispielsweise sind solche wasserlöslichen amphoteren Copolymerisate bevorzugt, die durch Copolymerisieren von
a) wenigstens einem N-Vinylcarbonsäureamid der allgemeinen Formel 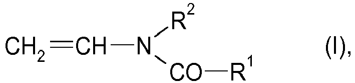 worin R¹ und R² unabhängig voneinander für H oder C₁- bis C₆-Alkyl stehen,
b) wenigstens einem Monomer aus der Gruppe (b2), das ausgewählt ist aus monoethylenisch ungesättigten Carbonsäuren mit 3 bis 8 C-Atomen und den wasserlöslichen Salzen wie Alkalimetall-, Erdalkalimetall- und Ammoniumsalzen dieser Carbonsäuren,
c) gegebenenfalls wenigstens einem von den Komponenten (a) und (b) verschiedenen monoethylenisch ungesättigten Monomer, und
d) gegebenenfalls wenigstens eine Verbindung, die mindestens zwei ethylenisch ungesättigte Doppelbindungen im Molekül aufweist,
und anschließende teilweise oder vollständige Hydrolyse der Gruppen -CO-R¹ aus den in das Copolymerisat einpolymerisierten Monomeren (a) erhältlich sind.

Besonders bevorzugt sind solche wasserlöslichen, amphoteren Copolymerisate, die erhältlich sind durch Copolymerisieren von
a) N-Vinylformamid,
b) Acrylsäure, Methacrylsäure und/oder deren Allkalimetall- oder Ammoniumsalzen, und
c) gegebenenfalls anderen monoethylenisch ungesättigten Monomeren und anschließende Abspaltung der -CO-R¹-Gruppe aus den Copolymerisaten.

Die Hydrolyse der nach dem oben beschriebenen Verfahren erhaltenen Polymere erfolgt nach bekannten Verfahren durch Einwirkung von Säuren, Basen oder Enzymen, beispielsweise Salzsäure, Natronlauge oder Kalilauge. Hierbei entstehen aus den einpolymerisierten Monomeren (a) der oben angegebenen Formel (I) durch Abspaltung der -CO-R¹-Gruppe Copolymerisate, die Vinylamineinheiten (VI) und/oder Amidineinheiten (II - V) 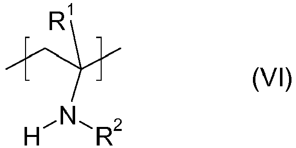 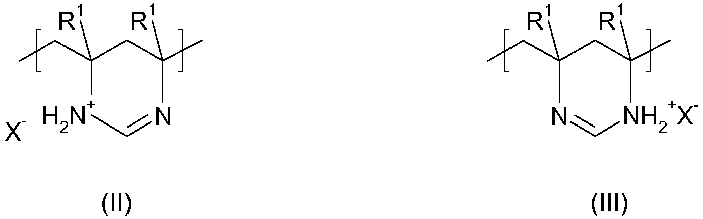 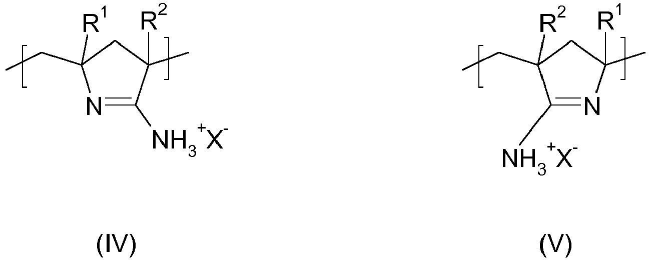 wobei in den Amidineinheiten (II) bis (V) X⁻ jeweils ein Anion bedeutet und die Substituenten R¹ und R² in den Formeln II - VI jeweils die in Formel I angegebene Bedeutung haben.

Das ursprünglich anionische Copolymerisat erhält durch die Hydrolyse kationische Gruppen und wird somit amphoter.

Die Amidineinheiten (II) und (III) entstehen durch Reaktion benachbarter Vinylaminheiten der Formel (VI) mit Vinylformamideinheiten bzw. durch Reaktion benachbarter Vinylamineinheiten der Formel (VI) mit Acrylnitril- oder Methacrylnitrilgruppen.

Die Hydrolyse der Copolymeren ist beispielsweise in EP-B-0 672 212 auf Seite 4, Zeilen 38 - 58 und auf Seite 5, Zeilen 1 - 25 und in den Beispielen von EP 528 409 detailliert offenbart. Bevorzugt werden hydrolysierte Copolymerisate eingesetzt, bei der die Hydrolyse in Gegenwart von Basen, bevorzugt in Gegenwart von Natronlauge, durchgeführt wurde.

Von besonderer technischer Bedeutung sind diejenigen amphoteren Copolymere, die als Komponente (i) N-Vinylformamid einpolymerisiert enthalten.

Das Verhältnis B von kationischen zu anionischen Gruppen im hydrolysierten Copolymerisat beträgt vorzugsweise 12 : 1 bis 1 : 12, insbesondere 7 : 1 bis 1 : 7.

Die Herstellung der wasserlöslichen amphoteren Copolymerisate, erfolgt nach üblichen, dem Fachmann bekannten Verfahren. Geeignete Verfahren sind z.B. in der EP-A-0 251 182, WO-A-94/13882 und EP-B-0 672 212 beschrieben, worauf hier Bezug genommen wird. Weiterhin wird auf die Herstellung der in WO-A-04/087818 und WO-A-05/012637 beschriebenen wasserlöslichen amphoteren Copolymerisate Bezug genommen.

Die Herstellung der wasserlöslichen amphoteren Copolymerisate kann durch Lösungs-, Fällungs-, Suspensions- oder Emulsionspolymerisation erfolgen. Bevorzugt ist die Lösungspolymerisation in wässrigen Medien. Geeignete wässrige Medien sind Wasser und Gemische aus Wasser und mindestens einem wassermischbaren Lösungsmittel, z.B. einem Alkohol, wie Methanol, Ethanol, n-Propanol, Isopropanol etc.

Die Polymerisationstemperaturen liegen vorzugsweise in einem Bereich von etwa 30 bis 200 °C, besonders bevorzugt 40 bis 110 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 0,1 und 5 bar.

Die Säuregruppen-haltigen Monomere (b) werden vorzugsweise in der Salzform eingesetzt. Der pH-Wert wird zur Copolymerisation vorzugsweise auf einen Wert im Bereich von 6 bis 9 eingestellt. Durch Einsatz eines üblichen Puffers oder durch Messung des pH-Werts und entsprechende Zugabe von Säure oder Base kann der pH-Wert während der Polymerisation konstant gehalten werden.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-amylperoxid, tert.-Butylhydroperoxid, Azo-bis-isobutyronitril, Azo-bis-(2-amidonopropan)dihydrochlorid oder 2-2'-Azo-bis-(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/CuI.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure, Natriumhypophosphit, Ameisensäure oder Dodecylmercaptan sowie Tribromchlormethan oder andere Verbindungen, die regelnd auf das Molekulargewicht der erhaltenen Polymerisate wirken, eingesetzt werden.

Die Molmasse der wasserlöslichen amphoteren Copolymerisate beträgt beispielsweise mindestens 10 000, vorzugsweise mindestens 100 000 Dalton und insbesondere mindestens 500 000 Dalton. Die Molmassen der Copolymerisate betragen dann z.B. 10 000 bis 10 Millionen, vorzugsweise 100 000 bis 5 Millionen (z.B. bestimmt durch Lichtstreuung). Dieser Molmassenbereich entspricht beispielsweise K-Werten von 5 bis 300, vorzugsweise 10 bis 250 (bestimmt nach H. Fikentscher in 5%iger wässriger Kochsalzlösung bei 25 °C und einer Polymerkonzentration von 0,1 Gew.-%).

Die wasserlöslichen, amphoteren Copolymerisate können eine anionische oder eine kationische Überschußladung tragen oder auch elektrisch neutral sein, wenn gleich viele anionische und kationische Gruppen im Copolymerisat vorliegen.

Die wasserlöslichen, amphoteren Copolymerisate werden zur Vorbehandlung von nativen und zurückgewonnenen Fasern eingesetzt. Verwendung finden können alle in der Papierindustrie üblicherweise eingesetzte Fasern aus Nadel- und Laubhölzern z.B. Holzstoff, gebleichter und ungebleichter Zellstoff sowie Papierstoffe aus allen Einjahrespflanzen. Zu Holzstoff gehören beispielsweise Holzschliff, thermomechanischer Stoff (TMP), chemo-thermomechanischer Stoff (CTMP), Druckschliff, Halbzellstoff, Hochausbeute-Zellstoff und Refiner Mechanical Pulp (RMP). Als Zellstoff kommen beispielsweise Sulfat-, Sulfit- und Natronzellstoffe in Betracht. Vorzugsweise verwendet man ungebleichten Zellstoff, der auch als ungebleichter Kraftzellstoff bezeichnet wird. Geeignete Einjahrespflanzen zur Herstellung von Papierstoffen sind beispielsweise Reis, Weizen, Zuckerrohr und Kenaf. Zur Herstellung der Pulpen kann auch Altpapier verwendet werden, das entweder allein oder in Mischung mit anderen Faserstoffen eingesetzt wird. Das Altpapiers kann beispielsweise aus einem Deinking-Prozess stammen. Es ist aber nicht erforderlich, dass das einzusetzende Altpapier einem solchen Prozess unterworfen wird. Weiterhin kann man auch von Fasermischungen aus einem Primärstoff und zurückgeführtem gestrichenem Ausschuss ausgehen.

Die Behandlung der Cellulosefasern wird in wässriger Suspension durchgeführt, vorzugsweise in Abwesenheit anderer Prozesschemikalien, die üblicherweise bei der Papierherstellung eingesetzt werden. Sie erfolgt bevorzugt im Papierherstellungprozess, indem man zu einer wässrigen Suspension von Fasern mindestens ein wasserlösliches, amphoteres Amidingruppen enthaltendes Copolymer zusetzt. Besonders bevorzugt ist eine Verfahrensvariante, bei der man ein wasserlösliches, amphoteres Amidingruppen enthaltendes Copolymerisat der Fasersuspension zu einem Zeitpunkt zusetzt, bevor weitere übliche Prozesschemikalien für die Papierherstellung dosiert werden. Die wasserlöslichen, amphoteren Copolymeren werden im Papierherstellungsprozess einem Dickstoff zugesetzt. In einer weiteren bevorzugten Variante setzt man die wasserlöslichen, amphoteren Copolymerisate einem Dickstoff zu, bevor man zum Papierstoff einen Füllstoff zugibt.

Typische Aufwandmengen sind vorzugsweise 0,3 bis 4 kg mindestens eines wasserlöslichen, amphoteren Copolymerisats, pro Tonne eines trockenen Faserstoffs. In den meisten Fällen betragen die eingesetzten Mengen an amphoterem Copolymerisat 0,5 bis 2,5 kg Polymer (fest), bezogen pro Tonne trockenen Faserstoff.

Die Einwirkzeit des amphoteren, Amidingruppen enthaltenden Polymeren auf einen reinen Faserstoff bzw. Gesamtstoff nach der Dosierung bis zur Blattbildung beträgt beispielsweise 0,5 Sekunden bis 2 Stunden, bevorzugt 1,0 Sekunden bis 15 Minuten, besonders bevorzugt 2 bis 20 Sekunden.

In einer bevorzugten Ausgestaltung der Erfindung erfolgt die Verwendung der oben beschriebenen wasserlöslichen, amphoteren Copolymerisate durch eine Vorbehandlung einer wässrigen Faserstoffsuspension in einem Papierherstellungsprozess bevor man zum Papierstoff andere übliche Prozesschemikalien dosiert.

Bei dem erfindungsgemäßen Verfahren können die üblicherweise bei der Papierherstellung verwendeten Prozesschemikalien in den üblichen Mengen eingesetzt werden, z.B. Retentionsmittel, Entwässerungsmittel, andere Trockenverfestiger wie beispielsweise Stärke, Pigmente, Füllstoffe, optische Aufheller, Entschäumer, Biozide und Papierfarbstoffe. Diese Stoffe werden dem Papierstoff vorzugsweise erst nach der erfindungsgemäßen Behandlung des Faserstoffs zugesetzt.

Die K-Werte der Copolymerisate wurden nach H. Fikentscher, Cellulose-Chemie, Band13, 48-64 und 71-74 (1932) in 5,0 %iger wässriger Kochsalzlösung bei 25°C, einem pH von 7 und einer Polymerkonzentration von 0,1 Gew.-% bestimmt.

Der Hydrolysegrad der Polymerisat kann durch enzymatische Analyse der bei der Hydrolyse freigesetzten Ameisensäure/Formiate bestimmt werden.

Die strukturelle Zusammensetzung der Polymerisate wurde aus der eingesetzten Monomerenmischung, dem Hydrolysegrad und dem mittels 13C-NMR-Spektrokopie bestimmten Verhältnis von Vinylamin/Amidin errechnet.

Die Prozentangaben in den Beispielen sind Gewichtsprozent, sofern nichts anderes angegeben ist.

### Beispiele

### Herstellung der Copolymere

### Polymer I

Zur Herstellung von Zulauf 1 wurden in einem Becherglas 150 g Eis vorgelegt, zuerst 69,2 g Acrylsäure und dann unter Rühren 384 g einer 10%igen Natronlauge zugegeben. Nach beendeter Neutralisation hatte die Lösung einen pH von 6,2. Anschließend wurden 103,4 g N-vinylformamid untergemischt.
Als Zulauf 2 wurden 0,52 g 2,2'-Azobis(2-methylpropionamidin)dihydrochlorid in 51 g Wasser bei Raumtemperatur gelöst.
Als Zulauf 3 wurden 0,34 g 2,2'-Azobis(2-methylpropionamidin)dihydrochlorid in 34,1 g Wasser bei Raumtemperatur gelöst.

In einer 2 l Glasapparatur mit Ankerrührer, Rückflusskühler, Innenthermometer und Stickstoffeinleitungsrohr wurden 400,0 g destilliertes Wasser und 2,1 g 75 %ige Phosphorsäure vorgelegt. Bei einer Drehzahl von 100 UpM wurden 8,0 g einer 10 %igen Natronlauge so zugegeben, dass ein pH von 6,5 erreicht wurde. In die Vorlage wurde für eine halbe Stunde Stickstoff mit 10l/h eingeleitet, um den vorhandenen Sauerstoff zu entfernen. Man erwärmte die Vorlage auf 74 °C. Dann wurden die Zuläufe 1 und 2 gleichzeitig gestartet. Bei konstanten 74 °C wurde der Zulauf 1 in 2 h und der Zulauf 2 in 3 Stunden zugefahren. Nach Beendigung der Zugabe von Zulauf 2 wurde das Reaktionsgemisch eine weitere Stunde bei 74 °C nachpolymerisiert. Dann gab man Zulauf 3 auf einmal zu und unterwarf die Mischung anschließend weitere 2 h bei 74 °C einer Nachpolymerisation. Schließlich wurden 403 g Wasser zugegeben und der Ansatz auf Raumtemperatur abgekühlt. Man erhielt eine leicht gelbe, viskose Lösung mit einem Festgehalt von 12,4 %. Der K-Wert des Terpolymeren betrug 115.

528,0 g des vorstehenden Produktes wurden in einem 1 l Dreihalskolben mit Blattrührer, Innenthermometer, Tropftrichter und Rückflusskühler bei einer Rührerdrehzahl von 80 UpM auf 80 °C erhitzt. Nachdem diese Temperatur erreicht war, wurden zuerst 2,4 g einer 25 %igen wässrigen Natriumdisulfitlösung und anschließend 40,4 g einer 25 %igen wässrigen Natronlauge so zugegeben, dass sie sich gut untermischten. Das Reaktionsgemisch wurde 3 Stunden bei 80°C gehalten und dann auf Raumtemperatur abgekühlt. Durch die langsame Zugabe von ca. 17,7 g konz. Salzsäure wurde der pH auf 8,6 eingestellt. Man erhielt eine viskose, farblose, leicht trübe Lösung mit einem Festgehalt von 13,6 %. Der Hydrolysegrad der einpolymerisierten Vinylformamideinheiten betrug 50 mol%.

Das erhaltene Polymer I hatte folgende Struktureinheiten:

| | |
|---|---|
| Vinylformamid: | 30 mol % |
| Vinylamin | 16 mol % |
| Amidin: | 14 mol % |
| Natriumacrylat: | 40 mol % |

### Polymer II

Zur Herstellung von Zulauf 1 wurden in einem Becherglas 44,9 g Wasser und 105 g Eis vorgelegt. Dann wurden 49,8 g Acrylsäure und unter Rühren 264,6 g einer 10 %igen wässrigen Natronlauge zugegeben. Nach beendeter Neutralisation hatte die Lösung einen pH von 6,5. Anschließend wurden 115,8 g N-vinylformamid untergemischt.
Als Zulauf 2 wurden 0,63 g 2,2'-Azobis(2-methylpropionamidin)dihydrochlorid in 50 g Wasser bei Raumtemperatur gelöst.
Als Zulauf 3 wurden 0,16 g 2,2'-Azobis(2-methylpropionamidin)dihydrochlorid in 8,8 g Wasser bei Raumtemperatur gelöst.

In einer 2 l Glasapparatur mit Ankerrührer, Rückflusskühler, Innenthermometer und Stickstoffeinleitungsrohr wurden 480,0 g destilliertes Wasser und 1,3 g 75 %ige Phosphorsäure vorgelegt. Bei einer Drehzahl von 100 UpM wurden dann 4,9 g einer 10 %i-gen wäßrigen Natronlauge so zugegeben, dass ein pH von 6,6 erreicht wurde. In die Vorlage leitete man für eine halbe Stunde Stickstoff mit 10l/h ein, um den vorhandenen Sauerstoff zu entfernen. Die Vorlage wurde auf 73 °C erwärmt. Dann wurden die Zuläufe 1 und 2 gleichzeitig gestartet. Bei konstanten 73°C wurde der Zulauf 1 in 2 h und der Zulauf 2 in 3 Stunden zugefahren. Nach Beendigung der Zugabe von Zulauf 2 wurde eine weitere Stunde bei 73 °C nachpolymerisiert. Dann gab man Zulauf 3 auf einmal zu und unterwarf das Reaktionsgemisch anschließend 2 h bei 73 °C einer Nachpolymerisation. Schließlich wurden 373 g Wasser zugegeben und der Ansatz auf Raumtemperatur abgekühlt. Man erhielt eine fast farblose, viskose Lösung mit einem Festgehalt von 12,7 %. Der K-Wert des Polymeren betrug 119.

576,0 g des vorstehenden Produktes wurden in einem 1 l Dreihalskolben mit Blattrührer, Innenthermometer, Tropftrichter und Rückflusskühler bei einer Rührerdrehzahl von 80 UpM auf 80 °C erhitzt. Man gab zuerst 3,3 g einer 25 %igen wässrigen Natriumdisulfitlösung und anschließend 32,4 g einer 25 %igen wässrigen Natronlauge so zu, dass sie sich gut untermischten. Das Reaktionsgemisch wurde 3 Stunden bei 80 °C gehalten und dann auf Raumtemperatur abgekühlt. Durch die langsame Zugabe von ca. 13,6 g konz. Salzsäure wurde der pH auf 9,0 eingestellt. Man erhielt eine viskose, schwach gelbliche, leicht trübe Lösung mit einem Festgehalt von 10,9 % erhalten. Der Hydrolysegrad betrug 32 mol%, bezogen auf das einpolymerisierte N-Vinylformamid.

Das erhaltene Polymer II enthielt folgende Struktureinheiten:

| | |
|---|---|
| Vinylformamid: | 48 mol % |
| Vinylamin | 9 mol % |
| Amidin: | 13 mol % |
| Natriumacrylat: | 30 mol % |

### Polymer III

Dieses Polymer wurde nach den Angaben in Beispiel 1 der JP-A-08059740 hergestellt. Das so erhaltene Polymer III hatte einen K-Wert von 65 und enthielt folgende Struktureinheiten:

| | |
|---|---|
| Vinylformamid: | 20 mol % |
| Vinylamin | 10 mol % |
| Amidin: | 35 mol % |
| Natriumacrylat: | 05 mol % |
| Acrylnitril | 30 mol % |

### Polymer IV (hergestellt gemäß WO-A-05/012637, Beispiel 1)

In einer 2 l Glasapparatur mit Ankerrührer, Rückflusskühler, Innenthermometer und Stickstoffeinleitungsrohr wurden 1339,0 g destilliertes Wasser, 3,8 g 75 %ige Phosphorsäure, 202,0 g 25 %ige Natriumvinylsulfonatlösung in Wasser, und 69,9 g Acrylsäure bei einer Drehzahl von 100 UpM gemischt. Durch Zutropfen von ca. 84 g einer 50 %igen wässrigen Natronlauge wurde der pH auf 6,8 eingestellt. Dann wurden 181,4 g Vinylformamid zugegeben. Unter Einleiten von Stickstoff wurde die Mischung auf 62 °C erhitzt. Nach Erreichen dieser Temperatur wurden innerhalb von 5 min 20,0 g einer 1,5 %igen wässrigen Lösung von 2,2'-Azobis(2-methylpropionamidin)dihydrochlorid zugegeben. Weitere 81,5 g einer 1,5 %igen wässrigen Lösung von 2,2'-Azobis(2-methylpropionamidin)dihydrochlorid wurden innerhalb von 4 Stunden zugefahren. Nach einer Polymerisationszeit von 3 Stunden wurdd die Temperatur auf 75°C erhöht. Nach einer weiteren Stunde bei 75 °C wurden 0,75 g 2,2'-Azobis(2-methylpropionamidin)dihydrochlorid in 20,0 g destilliertem Wasser zugesetzt und 2 Stunden bei 75 °C nachpolymerisiert. Nach dem Abkühlen auf Raumtemperatur wurde eine leicht trübe, farblose, stark viskose Lösung mit einem Festgehalt von 18,6 % erhalten. Der K-Wert des Terpolymeren betrug 122.

500,0 g des vorstehenden Produktes wurden in einem 1 l Dreihalskolben mit Blattrührer, Innenthermometer, Tropftrichter und Rückflusskühler bei einer Rührerdrehzahl 80 UpM auf 80 °C erhitzt. Man dosierte zuerst 6,3 g einer 25 %igen wässrigen Natriumdisulfitlösung und anschließend 60,5 g einer 25 %igen wässrigen Natronlauge so zu, dass sich die zugesetzten Komponenten gut untermischten. Das Reaktionsgemisch wurde 3 Stunden bei 80 °C gehalten und dann auf Raumtemperatur abgekühlt. Durch die langsame Zugabe von ca. 31 g konz. Salzsäure wurde der pH auf 7,2 eingestellt. Anschließend gab man 234,0 g destilliertes Wasser zur Verdünnung des Reaktionsgemisches zu. Nach dem Abkühlen auf Raumtemperatur erhielt man eine viskose, farblose, leicht trübe Lösung mit einem Feststoffgehalt von 15,0 % erhalten. Der Hydrolysegrad der einpolymerisierten Vinylformamideinheiten betrug 59 mol%.

Das erhaltene Polymer IV enthielt folgende Struktureinheiten:

| | |
|---|---|
| Vinylformamid: | 18 mol % |
| Vinylamin | 21 mol% |
| Amidin: | 22 mol % |
| Natriumvinylsulfonat: | 11 mol % |
| Natriumacrylat: | 28 mol % |

Prüfung der oben beschriebenen Polymeren I bis IV als Mittel zur Erhöhung der initialen Nassgefügefestigkeit von Papier

### Beispiele 1 bis 4 (nicht erfindungsgemäß)

Eine Mischung aus gebleichtem Birkensulfat und gebleichtem Kiefernsulfit wurde im Verhältnis von 70/30 bei einer Feststoffkonzentration von 4 % im Laborpulper stippenfrei aufgeschlagen, bis ein Mahlgrad von 30° SR erreicht war. Dem aufgeschlagenen Stoff wurde anschließend ein optischer Aufheller (Blankophor® PSG) sowie eine aufgeschlossene kationische Stärke (HiCat® 5163 A) zugegeben. Der Aufschluß der kationischen Stärke erfolgte als 10 %ige Stärkeslurry in einem Jet-Kocher bei 130°C und 1 Minute Verweilzeit. Die Dosiermenge des optischen Aufhellers betrug 0,5 % Handelsware bezogen auf den Trockengehalt der Papierstoffsuspension. Die Dosiermenge der kationischen Stärke betrug 0,5 % Stärke, bezogen auf den Trockengehalt der Papierstoffsuspension. Die Feststoffkonzentration der Faserstoffsuspension nach Zugabe von Stärke und optischem Aufheller betrug 3,7 %.

Vier Bechergläser wurden mit jeweils 50g der oben beschriebenen Faserstoffsuspension befüllt und anschließend durch Zugabe von Wasser auf eine Feststoffkonzentration von jeweils 0,35 % verdünnt. Zu jeder dieser Proben dosierte man jeweils eines der oben beschriebenen Polymeren I bis IV als 1 %-ige wässrige Lösung unter leichtem Rühren der Faserstoffsuspension. Die zugegebene Menge betrug 0,3 g. Danach wurde ein Füllstoff in Form eines handelsüblichen Carbonat-Pigments (GCC, Hydrocarb® 60, der Firma Omya) zugesetzt. Die Pigment-Slurry wurde vor der Zugabe zum Faserstoff auf einen Feststoffgehalt von 20 % verdünnt. Die zugegebene Menge der Füllstoffslurry wurde in mehreren Vorversuchen so eingestellt, dass der Füllstoffgehalt in den daran anschließend gebildeten Laborblättern etwa 20 % betrug.

### Beispiele 5 bis 8 (nicht erfindungsgemäß)

Eine Mischung aus gebleichtem Birkensulfat und gebleichtem Kiefernsulfit wurde im Verhältnis von 70/30 bei einer Feststoffkonzentration von 4 % im Laborpulper stippenfrei aufgeschlagen, bis ein Mahlgrad von 30° SR erreicht war. Dem aufgeschlagenen Stoff wurde anschließend ein optischer Aufheller (Blankophor® PSG) sowie eine aufgeschlossene kationische Stärke (HiCat® 5163 A) zugegeben. Der Aufschluss der kationischen Stärke erfolgte als 10 %ige Stärkeslurry in einem Jet-Kocher bei 130°C und 1 Minute Verweilzeit. Die Dosiermenge des optischen Aufhellers betrug 0,5 % Handelsware bezogen auf den Trockengehalt der Papierstoffsuspension. Die Dosiermenge der aufgeschlossenen kationischen Stärke betrug 0,5 % Stärke, bezogen auf den Trockengehalt der Papierstoffsuspension. Die Feststoffkonzentration der Faserstoffsuspension nach Zugabe von Stärke und optischem Aufheller betrug 3,7%.
Vier Bechergläser wurden mit jeweils 50g der oben beschriebenen Faserstoffsuspension befüllt. Zu jeder dieser Proben dosierte man jeweils eines der oben beschriebenen Polymeren I bis IV als 1 %-ige wässrige Lösung unter leichtem Rühren der Faserstoffsuspension. Die zugegebene Menge betrug 0,3 g. Der mit dem Polymer behandelte Stoff wurde danach jeweils durch die Zugabe von Wasser auf eine Feststoffkonzentration von 0,35 % verdünnt. Dann dosierte man einen Füllstoff in Form eines handelsüblichen Carbonat-Pigments (GCC, Hydrocarb 60, der Firma Omya). Die Pigment-Slurry wurde vor der Zugabe zum Faserstoff auf einen Feststoffgehalt von 20% verdünnt. Die zugegebene Menge der Füllstoffslurry wurde in mehreren Vorversuchen so eingestellt, dass der Füllstoffgehalt in den daran anschließend gebildeten Laborblättern etwa 20 % betrug.

### Beispiele 9 bis 12 (nicht erfindungsgemäß)

Eine Mischung aus gebleichtem Birkensulfat und gebleichtem Kiefernsulfit wurde im Verhältnis von 70/30 bei einer Feststoffkonzentration von 4 % im Laborpulper stippenfrei aufgeschlagen, bis ein Mahlgrad von 30° SR erreicht war. Dem aufgeschlagenen Stoff wurde anschließend ein optischer Aufheller (Blankophor® PSG) sowie eine aufgeschlossene kationische Stärke (HiCat® 5163 A) zugegeben. Der Aufschluß der kationischen Stärke erfolgte als 10 %ige wässrige Stärkeslurry in einem Jet-Kocher bei 130 °C und 1 Minute Verweilzeit. Die Dosiermenge des optischen Aufhellers betrug 0,5 % Handelsware bezogen auf den Trockengehalt der Papierstoffsuspension. Die Dosiermenge der kationischen Stärke betrug 0,5 % Stärke, bezogen auf den Trockengehalt der Papierstoffsuspension. Die Feststoffkonzentration der Faserstoffsuspension nach Zugabe von Stärke und optischen Aufheller betrug 3,7%.

Vier Bechergläser wurden mit jeweils 50g der oben beschriebenen Faserstoffsuspension befüllt. Diese Suspensionen wurden anschließend durch Zugabe von Wasser auf eine Feststoffkonzentration von jeweils 0,35 % verdünnt. Danach wurde ein Füllstoff in Form eines handelsüblichen Carbonat-Pigments (GCC, Hydrocarb 60, der Firma Omya) zugesetzt. Die wässrige Pigment-Slurry wurde vor der Zugabe zum Faserstoff auf einen Feststoffgehalt von 20 % verdünnt. Die zugegebene Menge der Füllstoffslurry wurde in mehreren Vorversuchen so eingestellt, dass der Füllstoffgehalt der später noch zu bildenden Laborblättern etwa 20 % betrug. Nach der Zugabe der Füllstoffslurry wurden jeweils die Polymeren I bis IV als 1%-tige Lösung und unter leichtem Rühren der Faserstoffsuspension zugegeben. Die zugegebene Menge betrug jeweils 0,3g.

### Vergleichsbeispiel 1

Eine Mischung aus gebleichtem Birkensulfat und gebleichtem Kiefernsulfit wurde im Verhältnis von 70/30 bei einer Feststoffkonzentration von 4 % im Laborpulper stippenfrei aufgeschlagen, bis ein Mahlgrad von 30° SR erreicht war. Dem aufgeschlagenen Stoff wurde anschließend ein optischer Aufheller (Blankophor® PSG) sowie eine aufgeschlossene kationische Stärke (HiCat® 5163 A) zugegeben. Der Aufschluß der kationischen Stärke erfolgte als 10 %ige wässrige Stärkeslurry in einem Jet-Kocher bei 130 °C und 1 Minute Verweilzeit. Die Dosiermenge des optischen Aufhellers betrug 0,5 % Handelsware bezogen auf den Trockengehalt der Papierstoffsuspension. Die Dosiermenge der kationischen Stärke betrug 0,5 % Stärke, bezogen auf den Trockengehalt der Papierstoffsuspension. Die Feststoffkonzentration der Faserstoffsuspension nach Zugabe von Stärke und optischem Aufheller betrug 3,7 %.

50g der so hergestellten Faserstoffsuspension wurden in ein Becherglas eingefüllt. Der Stoff wurde durch die Zugabe von Wasser auf eine Feststoffkonzentration von 0,35 % verdünnt.
Danach gab man einen Füllstoff in Form eines handelsüblichen Carbonat-Pigments (GCC, Hydrocarb 60, der Firma Omya) zu. Die wässrige Pigment-Slurry wurde vor der Zugabe zum Faserstoff durch Zugabe von Wasser auf einen Feststoffgehalt von 20 % verdünnt. Die zugegebene Menge der Füllstoffslurry wurde in mehreren Vorversuchen so eingestellt, dass der Füllstoffgehalt der später zu bildenden Laborblättern etwa 20 % betrug.

### Herstellung von Laborblättern und Bestimmung der initialen Nassgefügefestigkeit

Die in den Beispielen 1 bis 12 sowie in dem Vergleichsbeispiel 1 beschriebenen Suspensionen wurden jeweils zwei Minuten nach dem letzten Zugabeschritt auf einem Rapid-Köthen-Blattbildner nach ISO 5269/2 zu Blättern einer Flächenmasse von 100 g/m² verarbeitet.

Die Bestimmung der initialen Nassgefügefestigkeit an dem nassen Papier erfolgte jeweils nach dem Voith-Verfahren (vgl. M. Schwarz und K. Bechtel "Initiale Gefügefestigkeit bei der Blattbildung", in Wochenblatt für Papierfabrikation 131, Seiten 950 - 957 (2003) Nr. 16. Dazu wurden die nassen Blätter vom nassen Siebrahmen des Rapid-Köthen Blattbildners auf eine Kunstoffunterlage abgeschlagen und auf eine Schneideunterlage übertragen. Anschließend wurden Probestreifen mit einer definierten Länge und Breite aus dem Blatt geschnitten. Unter konstantem Druck wurden diese solange gepresst, bis der gewünschte Trockengehalt erreicht war. Für die Untersuchungen der nach den oben angegebenen Beispielen erhaltenen Papierblätter wurden jeweils vier Trockengehalte im Bereich zwischen 42 % und 58 % eingestellt. Aus diesen Werten wurde mit Hilfe eines in der oben angegebenen Literaturstelle beschriebenen mathematischen Anpassungsverfahrens die initiale Nassgefügefestigkeit bei 50 % Trockengehalt bestimmt. Die eigentliche Messung der initialen Nassgefügefestigkeit erfolgte an einer vertikalen Zugprüfmaschine mit einer speziellen Klemmeinrichtung. Die in der Zugmaschine bestimmte Kraft wurde in den Flächenmasseunabhängigen sogenannten INF-Index umgerechnet. Eine genaue Beschreibung der Klemmeinrichtung, des Messablaufs, der Bestimmung des Trockengehaltes im Papier und der Datenverarbeitung vgl. die oben angegebenen Literaturstelle.

Die Ergebnisse der Prüfungen sind in Tabelle 1 wiedergegeben

**Tabelle 1**

| | INF-Index [Nm/g] |
|---|---|
| Beispiel 1¹⁾ | 3,0 |
| Beispiel 2¹⁾ | 2,9 |
| Beispiel 3¹⁾ | 2,9 |
| Beispiel 4¹⁾ | 3,4 |
| Beispiel 5¹⁾ | 2,2 |
| Beispiel 6¹⁾ | 2,2 |
| Beispiel 7¹⁾ | 2,1 |
| Beispiel 8¹⁾ | 2,4 |
| Beispiel 9¹⁾ | 2,3 |
| Beispiel 10¹⁾ | 2,4 |
| Beispiel 11¹⁾ | 2,5 |
| Beispiel 12¹⁾ | 2,6 |
| Vergleichsbeispiel 1 | 1,8 |

| | |
|---|---|
| ¹⁾: nicht erfindungsgemäss | |

## Patentansprüche

1. Verwendung von wasserlöslichen, amphoteren Copolymeren, die erhältlich sind durch Copolymerisieren von
a) wenigstens einem N-Vinylcarbonsäureamid der allgemeinen Formel 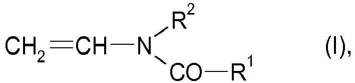 worin R¹ und R² unabhängig voneinander für H oder C₁- bis C₆-Alkyl stehen,
b) wenigstens einem Monomer, das ausgewählt ist aus der Gruppe bestehend aus
(b1) monoethylenisch ungesättigten Sulfonsäuren, Phosphonsäuren, Phosphorsäureestern und Derivaten davon, und
(b2) monoethylenisch ungesättigten Mono- und Dicarbonsäuren, deren Salzen und Dicarbonsäureanhydriden,
c) gegebenenfalls wenigstens einem von den Komponenten (a) und (b) verschiedenen monoethylenisch ungesättigten Monomer, und
d) gegebenenfalls wenigstens einer Verbindung, die mindestens zwei ethylenisch ungesättigte Doppelbindungen im Molekül aufweist,
mit der Maßgabe, dass das Monomerengemisch mindestens ein Monomer (b) mit mindestens einer freien Säuregruppe und/oder eine Säuregruppe in Salzform enthält,
und anschließende teilweise Hydrolyse der Gruppen -CO-R¹ aus den in das Copolymerisat einpolymerisierten Monomeren (a), und diese Copolymere
(i) 5 bis 70 Mol-% Vinylcarbonsäureamideinheiten,
(ii) 5 bis 45 Mol-% Einheiten von Acrylsäure, Methacrylsäure, Salzen und Gemischen davon,
und
(iii) 10 bis 60 Mol-% Vinylamineinheiten der Formel VI in Salzform 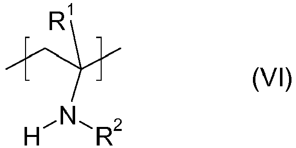 und/oder Amidineinheiten der Formel (II) und/oder (III) 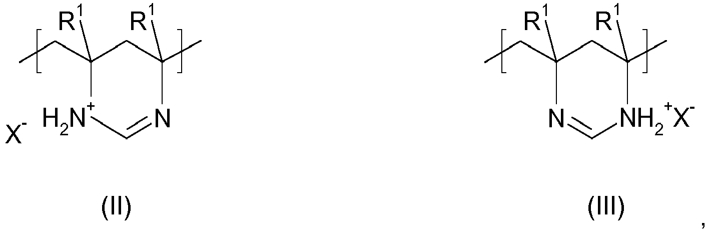 wobei in den Amidineinheiten (II) und (III) X-jeweils ein Anion bedeutet und die Substituenten R¹ und R² in den Formeln II, III und VI jeweils die in Formel I angegebene Bedeutung haben,
enthalten,
als Mittel zur Erhöhung der initialen Nassgefügefestigkeit von Papier,
**dadurch gekennzeichnet, dass** die wasserlöslichen, amphoteren Copolymeren in einer Menge von 0,03 bis 1,00 Gew.-%, bezogen auf trockenen Faserstoff, einem Dickstoff mit einer Faserkonzentration von > 15 g/l zugesetzt werden.

2. Verwendung nach Anspruch 1, **dadurch gekennzeichnet, dass** die zur Behandlung des Faserstoffs eingesetzten Copolymeren
(i) 5 bis 70 Mol-% Vinylcarbonsäureamideinheiten,
(ii) 3 bis 30 Mol-% Einheiten von monoethylenisch ungesättigten Sulfonsäuren, Phosphonsäuren und Salzen davon, und 5 bis 45 Mol-% Einheiten von Acrylsäure, Methacrylsäure, Salzen und Gemischen davon,
und
(iii) 10 bis 60 Mol-% Vinylamineinheiten der Formel VI in Salzform und/oder Amidineinheiten der Formel (II) und/oder (III)
enthalten.

3. Verwendung nach einem der Ansprüche 1 bis 2, **dadurch gekennzeichnet, dass** die wasserlöslichen, amphoteren Copolymeren einem Dickstoff vor Zugabe eines Füllstoffs zugesetzt werden.

## Claims

1. The use of water-soluble, amphoteric copolymers which are obtainable by copolymerization of
a) at least one N-vinylcarboxamide of the general formula 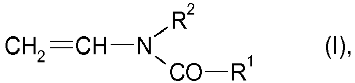 in which R¹ and R², independently of one another, are H or C₁- to C₆-alkyl,
b) at least one monomer which is selected from the group consisting of
(b1) monoethylenically unsaturated sulfonic acids, phosphonic acids, phosphoric acid esters and derivatives thereof, and
(b2) monoethylenically unsaturated mono- and dicarboxylic acids, the salts thereof and dicarboxylic anhydrides,
c) optionally, at least one monoethylenically unsaturated monomer differing from the components (a) and (b), and
d) optionally, at least one compound which has at least two ethylenically unsaturated double bonds in the molecule,
with the proviso that the monomer mixture comprises at least one monomer (b) having at least one free acid group and/or an acid group in salt form,
and subsequent partial hydrolysis of the groups -COR¹ from the monomers (a) incorporated in the form of copolymerized units into the copolymer, and said copolymers comprise
(i) from 5 to 70 mol% of vinylcarboxamide units,
(ii) from 5 to 45 mol% of units of acrylic acid, methacrylic acid, salts and mixtures thereof,
and
(iii) from 10 to 60 mol% of vinylamine units of the formula VI in salt form 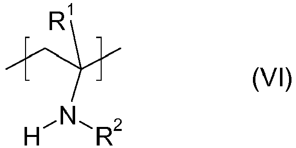 and/or amidine units of the formula (II) and or (III) 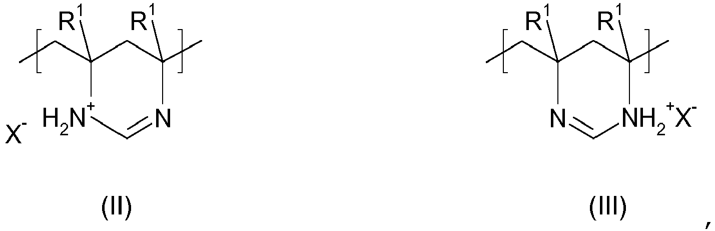
in the amidine units (II) and (III), X⁻ being in each case an anion and the substituents R¹ and R² in the formulae II, III and VI having in each case the meaning stated in formula I,
as agents for increasing the initial wet web strength of paper,
wherein the water-soluble, amphoteric copolymers are added in an amount of from 0.03 to 1.00% by weight, based on dry fiber, to a high-consistency stock having a fiber concentration of >15g/L.

2. The use according to claim 1, wherein the copolymers used for treating the fiber comprise
(i) from 5 to 70 mol% of vinylcarboxamide units,
(ii) from 3 to 30 mol% of units of monoethylenically unsaturated sulfonic acids, phosphonic acids and salts thereof and from 5 to 45 mol% of units of acrylic acid, methacrylic acid, salts and mixtures thereof,
and
(iii) from 10 to 60 mol% of vinylamine units of the formula VI in salt form and/or amidine units of the formula (II) and/or (III).

3. The use according to either of claims 1 and 2, wherein the water-soluble, amphoteric copolymers are added to a high-consistency stock before addition of a filler.

## Revendications

1. Utilisation de copolymères amphotères solubles dans l'eau, qui peuvent être obtenus par copolymérisation de :
a) au moins un amide d'acide N-vinylcarboxylique de formule générale 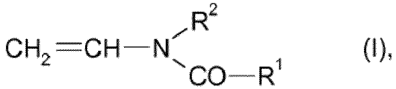 dans laquelle R¹ et R² représentent indépendamment l'un de l'autre H ou alkyle en C₁ à C₆,
b) au moins un monomère, qui est choisi dans le groupe constitué par :
(b1) les acides sulfoniques monoéthyléniquement insaturés, les acides phosphoniques, les esters d'acide phosphorique et leurs dérivés, et
(b2) les acides mono- et dicarboxyliques monoéthyléniquement insaturés, leurs sels et les anhydrides d'acides dicarboxyliques,
c) éventuellement au moins un monomère monoéthyléniquement insaturé différent des composants (a) et (b), et
d) éventuellement au moins un composé qui comprend au moins deux doubles liaisons éthyléniquement insaturées par molécule,
à condition que le mélange de monomères contienne au moins un monomère (b) contenant au moins un groupe acide libre et/ou un groupe acide sous forme saline,
puis hydrolyse partielle des groupes -CO-R¹ des monomères (a) copolymérisés dans le copolymère, ces copolymères contenant :
(i) 5 à 70 % en moles d'unités amide d'acide vinylcarboxylique,
(ii) 5 à 45 % en moles d'unités d'acide acrylique, d'acide méthacrylique, leurs sels et leurs mélanges,
et
(iii) 10 à 60 % en moles d'unités vinylamine de formule VI sous forme saline, 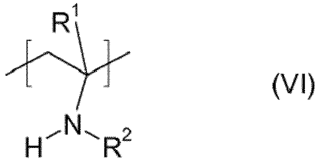 et/ou d'unités amidine de formule (II) et/ou (III) 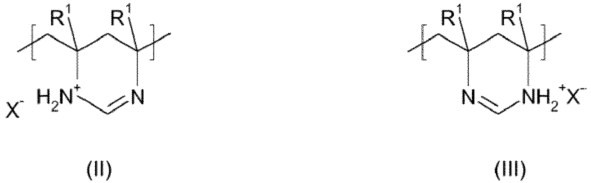 dans les unités amidine (II) et (III), X⁻ signifiant à chaque fois un anion et les substituants R¹ et R² dans les formules II, III et VI ayant chacun la signification indiquée pour la formule I,
en tant qu'agent pour augmenter la stabilité structurelle initiale à l'état humide de papier,
**caractérisée en ce que** les copolymères amphotères solubles dans l'eau sont ajoutés en une quantité de 0,03 à 1,00 % en poids, par rapport à la matière fibreuse sèche, à une matière épaisse ayant une concentration en fibres > 15 g/l.

2. Utilisation selon la revendication 1, **caractérisée en ce que** les copolymères utilisés pour le traitement de la matière fibreuse contiennent :
(i) 5 à 70 % en moles d'unités amide d'acide vinylcarboxylique,
(ii) 3 à 30 % en moles d'unités d'acides sulfoniques monoéthyléniquement insaturés, d'acides phosphoniques et leurs sels, et 5 à 45 % en moles d'unités d'acide acrylique, d'acide méthacrylique, leurs sels et leurs mélanges,
et
(iii) 10 à 60 % en moles d'unités vinylamine de formule VI sous forme saline et/ou d'unités amidine de formule (II) et/ou (III).

3. Utilisation selon l'une quelconque des revendications 1 à 2, **caractérisée en ce que** les copolymères amphotères solubles dans l'eau sont ajoutés à une matière épaisse avant l'ajout d'une charge.
